# EUROPEAN PATENT APPLICATION

(11) **EP 4 287 196 A1**
(43) Date of publication of application: **06.12.2023**
(21) Application number: 22176059.8
(22) Date of filing: 30.05.2022
(51) Int. Cl.: G16H 10/60

(54) **HEALTH DATABASE UPDATING**

(71) Applicant: Koninklijke Philips N.V., 5656 AG Eindhoven (NL)
(72) Inventor: JOHNSON, Mark Thomas, Eindhoven (NL); BULUT, Murtaza, Eindhoven (NL); VAN DER ZAAG, Pieter J., 5656AG Eindhoven (NL); MACINNES, Alyson, 5656AG Eindhoven (NL); BOUWMAN, Wilbert, 5656AG Eindhoven (NL)
(74) Representative: Philips Intellectual Property & Standards

(57) **Abstract**

Some embodiments are directed to a health database system. The health database system receives health data relating to a patient and medical updates from multiple data generating systems. The system determines if a received medical update is relevant for the patient, and, if so, store the medical update in a database storage associated with the determined patient.

## Description

### FIELD OF THE INVENTION

The presently disclosed subject matter relates to a health database system, health database method, and a computer readable medium.

### BACKGROUND OF THE INVENTION

Medical treatment is moving in a direction of increased specialization. For example, in fields such as oncology, diagnosis and treatment may be distributed-not just in hospitals, but across specialized centers for imaging, lab values, genomics, tissue and liquid biopsies.

This distribution may also involve multiple primary care professionals, such as the oncologist, pathologist, GP, nurses, etc. In such a distributed system, medical information is increasingly harder to access by the various parties that need such access.

Currently electronic medical record (EMR) systems are often used to store medical information. An EMR may be an electronic record of health-related information on an individual that can be created, gathered, managed, and consulted by authorized clinicians and staff within one health care organization. However, as distribution is expected to increase, an EMR may not suffice. An alternative to an EMR is a Personal Health Record (PHR). For example, patients may store and control their data in a PHR. In such a patient-controlled record, there may be many reasons to update the PHR, e.g., as new information comes in.

### SUMMARY OF THE INVENTION

There is a desire to improve the storage of medical data, and in particular its updating. For example, an embodiment of a health database system stores health data relating to a patient, received from multiple data generating systems, in a database. Medical updates that are received at the health database system are assessed for relevancy for a patient. If they are so determined, the medical update is stored in the database. For example, a medical update may comprise a care procedure or health data. The health data system is electronic, and may be an electronic device, in particular a computer, a server or the like.

A further aspect is a health database method. An embodiment of the method may be implemented on a computer as a computer implemented method, or in dedicated hardware, or in a combination of both. Executable code for an embodiment of the method may be stored on a computer program product. Examples of computer program products include memory devices, optical storage devices, integrated circuits, servers, online software, etc. Preferably, the computer program product comprises non-transitory program code stored on a computer readable medium for performing an embodiment of the method when said program product is executed on a computer.

In an embodiment, the computer program comprises computer program code adapted to perform all or part of the steps of an embodiment of the method when the computer program is run on a computer. Preferably, the computer program is embodied on a computer readable medium.

### BRIEF DESCRIPTION OF THE DRAWINGS

Further details, aspects, and embodiments will be described, by way of example, with reference to the drawings. Elements in the Figs. are illustrated for simplicity and clarity and have not necessarily been drawn to scale. In the Figs., elements which correspond to elements already described may have the same reference numerals. In the drawings,
Fig. 1a schematically shows an example of an embodiment of a health database system,
Fig. 1b schematically shows an example of an embodiment of a health information system,
Fig. 2a schematically shows an example of an embodiment of a health database system,
Fig. 2b schematically shows an example of an embodiment of a health database system,
Fig. 3 schematically shows an example of an embodiment of a health database method,
Fig. 4 schematically shows an example of an embodiment of a health database system,
Fig. 5a schematically shows an example of an embodiment of a health database system,
Fig. 5b schematically shows an example of an embodiment of a health database system,
Fig. 6 schematically shows an example of an embodiment of a health database method,
Fig. 7a schematically shows a computer readable medium having a writable part comprising a computer program according to an embodiment,
Fig. 7b schematically shows a representation of a processor system according to an embodiment.

### Reference signs list

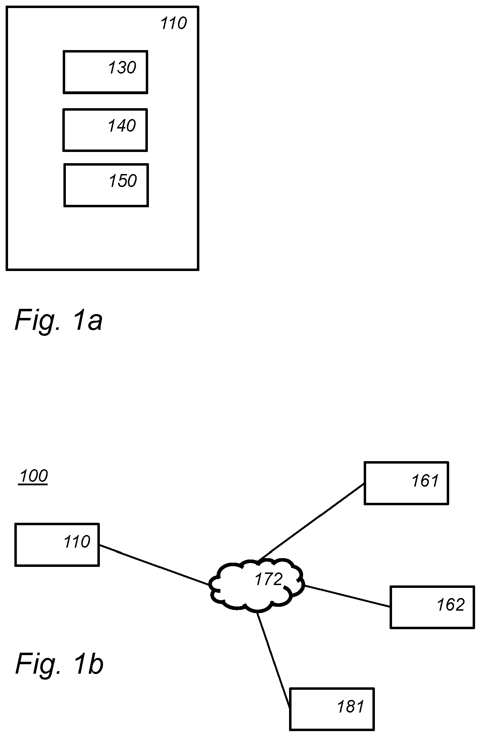

The following list of reference signs refers to Figs. 1a, 1b, 2a, 2b, 5a, 7a, 7b and is provided for facilitating the interpretation of the drawings and shall not be construed as limiting the claims.
- 100: a health information system
- 110: a health database system
- 130: a processor system
- 140: storage
- 150: communication interface
- 161, 162: data generating system
- 172: a computer network
- 181: a client device

- 221-223: a database
- 231-234: a data generating system
- 260: a care procedure database
- 270: a distributed ledger
- 271-273: a block
- 271.1-273.3: an information
- 521: a database
- 522-523: a protocol

- 1000, 1001: a computer readable medium
- 1010: a writable part
- 1020: a computer program
- 1110: integrated circuit(s)
- 1120: a processing unit
- 1122: a memory
- 1124: a dedicated integrated circuit
- 1126: a communication element
- 1130: an interconnect
- 1140: a processor system

### DETAILED DESCRIPTION OF EMBODIMENTS

While the presently disclosed subject matter is susceptible of embodiment in many different forms, there are shown in the drawings and will herein be described in detail one or more specific embodiments, with the understanding that the present disclosure is to be considered as exemplary of the principles of the presently disclosed subject matter and not intended to limit it to the specific embodiments shown and described.

In the following, for the sake of understanding, elements of embodiments are described in operation. However, it will be apparent that the respective elements are arranged to perform the functions being described as performed by them.

Further, the subject matter that is presently disclosed is not limited to the embodiments only, but also includes every other combination of features described herein or recited in mutually different dependent claims.

Fig. 1a schematically shows an example of an embodiment of a health database system 110. Health database system 110 may be part of a health information system 100. Fig. 1b schematically shows an example of an embodiment of a health information system 100.

Health database system 110 may be configured to store health data in association with a patient, and to determine if a received medical update is relevant for the patient. Health information system 100 may comprise one or more health database system(s) 110. Health information system 100 may comprise multiple data generating systems; shown are data generating system 161 and data generating system 162. Health database system 110 may be under the direct control of an end user, e.g., a patient. For example, health database system 110 may be implemented in a client device, e.g., a computer, desktop, laptop, smartphone, or the like. Health database system 110 may be implemented in the cloud, e.g., in a health database server, e.g., a computer, a server, a distributed computing system, or the like; health database system 110 may be accessed by an end-user by a client device, e.g., over a computer network. For example, client device 181 may be a computer, a laptop, a smartphone, or the like. The devices and/or systems are connected through a computer network 172, e.g., the Internet.

Health database system 110 may comprise a processor system 130, a storage 140, and a communication interface 150, e.g., a data interface. Storage 140 may be, e.g., electronic storage, magnetic storage, etc. The storage may comprise local storage, e.g., a local hard drive or electronic memory. Storage 140 may comprise non-local storage, e.g., cloud storage. In the latter case, storage 140 may comprise a storage interface to the non-local storage. Storage may comprise multiple discrete sub-storages together making up storage 140. Storage may comprise a volatile writable part, say a RAM, a non-volatile writable part, e.g., Flash, a non-volatile non-writable part, e.g., ROM.

Storage 140 may be non-transitory storage. For example, storage 140 may store data in the presence of power such as a volatile memory device, e.g., a Random Access Memory (RAM). For example, storage 140 may store data in the presence of power as well as outside the presence of power such as a non-volatile memory device, e.g., Flash memory.

System 110 may be implemented in a distributed system, e.g., a cloud implementation; for example, storage and/or processing may be performed at geographically different locations. System 110 may be implemented in a device, e.g., a computer, e.g., a server, located at one geographic location. System 110 may serve one or multiple end-users, e.g., patients, GP, and the like. Storage may be dedicated to one particular end-user, or may store data corresponding to multiple end-users.

System 110 may communicate internally, with other systems, with other devices, external storage, input devices, output devices, and/or one or more sensors over a computer network. The computer network may be an internet, an intranet, a LAN, a WLAN, etc. The computer network may be the Internet. System 110 comprise a connection interface which is arranged to communicate within system 100 or outside of system 100 as needed. For example, the connection interface may comprise a connector, e.g., a wired connector, e.g., an Ethernet connector, an optical connector, etc., or a wireless connector, e.g., an antenna, e.g., a Wi-Fi, 4G or 5G antenna.

The communication interface 150 may be used to send or receive digital data, e.g., health data, care procedure, medical update, health data update, care procedure update, and the like.

The execution of devices 110 may be implemented in a processor system. The devices 110 may comprise functional units to implement aspects of embodiments. The functional units may be part of the processor system. For example, functional units shown herein may be wholly or partially implemented in computer instructions that are stored in a storage of the device and executable by the processor system.

The processor system may comprise one or more processor circuits, e.g., microprocessors, CPUs, GPUs, etc. Devices 110 may comprise multiple processors. A processor circuit may be implemented in a distributed fashion, e.g., as multiple sub-processor circuits. For example, devices 110 may use cloud computing.

Typically, the health database system 110 comprises a microprocessor which executes appropriate software stored at the device; for example, that software may have been downloaded and/or stored in a corresponding memory, e.g., a volatile memory such as RAM or a non-volatile memory such as Flash.

Instead of using software to implement a function, device 110 may, in whole or in part, be implemented in programmable logic, e.g., as field-programmable gate array (FPGA). The devices may be implemented, in whole or in part, as a so-called application-specific integrated circuit (ASIC), e.g., an integrated circuit (IC) customized for their particular use. For example, the circuits may be implemented in CMOS, e.g., using a hardware description language such as Verilog, VHDL, etc. In particular, health database system 110 may comprise circuits, e.g., for cryptographic processing, and/or arithmetic processing.

In hybrid embodiments, functional units are implemented partially in hardware, e.g., as coprocessors, e.g., cryptographic coprocessors, artificial intelligence coprocessors, etc., and partially in software stored and executed on the device.

In an embodiment, health database system 110 comprises a database storage for storing at least health data in association with a patient. Health data relating to a patient may be received at system 110 from multiple data generating systems, e.g., over a data interface, e.g., implemented by interface 150. Typically, the database is a PHR comprising personal health records. Such a PHR is typically patient centric holding data from a multiplicity of data providers. The database may also comprise electronic medical records (EMR), and/or an electronic health records (EHR), though. Such an EMR or EHR is typically data provider centric holding data from a multiplicity of patients who interact with the data providers. Interestingly, the health data is received from multiple data generating sources, e.g., hospitals, laboratories, health practitioners, and the like. The database may relate to a single individual, but may also store information relevant to multiple persons.

Health data may be stored in association with a patient. Health data may comprise multiple types of data. For example, health data may be clinical information, including, diagnosis, test results, medical images, assessments, genomic data, lab values, and vital signs.

Health data may comprise data collected outside any particular hospital. For example, the health data may be collected by the patient him/herself, or by a device associated with the patient. For example, health data may be received from, e.g., a wearable sensor data, smart home sensor data, patient input, e.g., sleep dairies, nutrition diaries, input from family/caregivers, health related information extracted from social activity of the patient, and so on. Health data may also comprise medications, clinical notes, device information, procedure information, and document references. For example, a document reference may comprise a link to a medical guideline.

The database may store a care procedure, also in association with a patient. For example, a care procedure that has been selected as relevant to the patient, e.g., by a health practitioner. The application of the care procedure to this particular patient, e.g., obtained by applying a general care procedure to the patient and specifying general care procedure(s) for the patient by particular care procedure(s).

A care procedure, e.g., as stored in database 260, can refer to the care protocol, to one or more steps in the care protocols. A reference to a care protocol from a care procedure, may indicate a parent relation, e.g., for a care procedure that was taken from a care protocol. A care procedure may also refer, e.g., be associated with, another care protocol or care procedure. If a care procedure is linked to a care protocol, then when the protocols changes aspects of the procedures may also change. Instead of storing care procedures individually, e.g., as collected from care protocols, a care procedure may be stored as part of a stored care protocol.

The health data stored in association with a patient, may also comprise particular treatment and/or test steps that have been taken for a patient, in particular as indicated by an application of a care procedure to a patient, e.g., the care procedure may be stored in association with the patient. For example, a care procedure may indicate test and/or subject steps which are to be executed for a particular type of patient. Care procedures are frequently linked to care protocols, such that when care protocols change, aspects of the associated care procedures may change accordingly. The database may store the tests and/or treatments that have actually been performed for the patient; possibly in association with the corresponding elements of the care procedure. The database may also store interpretation data. This interpretation can be a human interpretation, e.g., performed by a health practitioner, but may also be a machine interpretation, e.g., an algorithm processing the patient data.

For example, a care procedure C1 may comprise elements X, Y, etc. The health database may store care procedure C1, as well as the actual elements that were applied to the patient, and possibly their results, e.g., elements X', Y'. In addition to the actually applied elements, the database may also store an association with the care procedure that caused them, e.g., a link between X' and C1, and Y' and C1. The number of actually applied elements may be longer or shorter, or may have elements not in the care procedure. If a care procedure is updated to C2, then the updated care procedure may be stored in the database. Furthermore, the elements X' and Y' which were applied because of C1 may have to be reconsidered. In many cases, such a reconsideration will not be significant. For example, C2 might not have changed in the area relevant to this patient; for example, C2 may still suggest X and Y. For example, even if C2 no longer recommends X and/or Y, if these are treatment steps that have already been performed, they cannot be changed retrospectively. On the other hand, some elements can be changed retrospectively; if element X or Y is a diagnosis say then the diagnosis may be changed. If X changes to Z, say, then other elements that rely on the diagnosis X now have to be reconsidered as well.

Other changes may also be important. For example, C2 may suggest a new element Z, that was not applied to the patient. For example, Z may be a new diagnosis, a new treatment. For example, a gene may be found to be relevant for this medical condition. In that case, element Z should be considered to application.

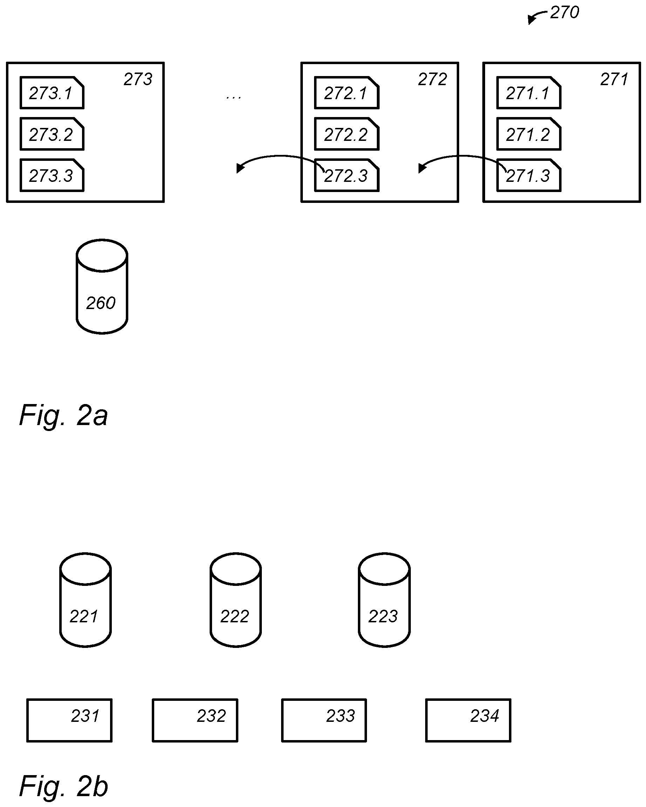

The database storage may comprise a distributed ledger, new tokens stored in said distributed ledger comprising health data and/or care procedures associated with a patient. Fig. 2a, discussed herein, gives an example of such a distributed ledger.

The database may store data about the patient and/or protocols or procedures relevant to the patient, received from multiple providers.

The health database system is configured to receive medical updates from the multiple data generating systems. A medical update may comprise a care procedure from one or more of the multiple data generating systems, in particular an update to a care procedure that was previously stored in the database in association with the patient, e.g., as relevant to the patient.

A care procedure generally applies to multiple patients. For example, a care procedure describes how to treat and/or diagnose in a particular situation. A care procedure may be part of a care protocol. For example, a care protocol may comprise one or more care procedures. In some cases the care procedure may be identical to the care protocol. An update in the care protocol may entail an update in the procedure that it comprises. A care protocol may be a written plan that specifies care procedures to be followed in defined situations.

For example, clinical guidelines may be interpreted by a hospital, and a customized care protocol may be created, e.g., specific to the hospital. In a typical hospital hundreds of protocols are available, e.g., relating to different cancer types. When new guidelines or new information become available these protocols are updated. Since, protocols can be specific to a hospital or a hospital chain, they may be updated on a weekly basis. A medical update may comprise an update to a care protocol, e.g., precipitated by an update of a medical guideline. Hence, the update in the care protocol may entail an update in procedure(s) that it comprises.

For example, a medical update may comprise receiving a care procedure update, the received care procedure comprising a new version identifier. Versioning may be used to determine if the received medical update is relevant for a patient. For example, the system may verify that the database storage stores an association between the patient and a care procedure that has a stored version identifier matching the new version identifier. The health database system processor may be configured to execute a version matching function to determine if the new version identifier and the stored identifier match.

Using a version number ensures that two version of the same thing are being compared. Version tracking works well, but a version identifier is not needed though. For example, two documents can be compared, e.g., using a file comparison algorithm, or a text based comparison algorithm. Natural language processing may be used to assess if the documents are different or the same. Other machine learning algorithms may be used instead. For example, a recurrent neural network may be trained to assess the difference between two documents, e.g., two care protocols, two care procedures or the like. The neural network may also be trained to indicate a relevance score of the difference for a particular patient.

A medical update may also comprise new medical information having a knock-on effect. For example, new information, may influence how the existing personal health record data, e.g., including patient data, protocol data, etc., may be re-interpreted.

The medical update, e.g., the new medical information, may or may not be linked to any of the previous protocols/procedures. The new information may be linked, in various ways, e.g., through an id, through keywords, a computer address, a unique resource locator, and the like. New information can have a knock-on effect by following one or more links associated with the new information. The update may or may not be linked to the procedure at the time it was carried out, though perhaps it should have been if known. Interpretation and re-interpretation refers both to interpretation by humans (e.g., clinicians) and by machines (e.g. algorithms making use of protocol data and/or patient data as input). An algorithm can be part of a medical database, such as, a PHR, EMR, or EHR. Below, the algorithm modification is discussed as a possible update.

A medical update may comprise an updated data interpretation, e.g., a diagnosis, made by an algorithm. For example, an algorithm may be updated, so that the result of the algorithm may be updated. For example, the updated result of the updated algorithm may be received at the health database system and stored in the database.

A medical update may comprise health data, e.g., a health data update, e.g., received from one or more of the multiple data generating systems. For example, the health data update may comprise an erratum, addendum, and/or amendment of medical data, e.g., test results.

A medical update may comprise a health data update of a previous health data. For example, the health database system may determine that the received medical update is relevant for a patient. This may comprise verifying that the database storage stores an association between the previous health data and a care procedure. For example, a health database system processor may be configured to determine if the health data update is relevant for the care procedure.

For example, the database may store for a particular patient that a diagnosis of cardiomyopathy was made on the basis of a chest x-ray, echocardiogram, and an electrocardiogram, or the like. If an update is received for any of these, say, a new electrocardiogram is received, or an amendment or the like, the system may look up the associated diagnosis and conclude that the diagnosis may need reinterpretation. The system may suggest that an ongoing treatment based on the earlier diagnosis may have to be stopped, and re-evaluated to account for potential changes in the diagnosis. The system may suggest that the ongoing treatment is paused until re-evaluation.

The health database system is configured to determine if a received medical update is relevant for the patient, and, if so, store the medical update in the database storage associated with the determined patient.

Some medical updates may be stored in any case, for example, health data associated with a particular patient may be stored in any case, associated with that patient. However, some medical updates may or may not be relevant for a patient. For example, a care protocol or procedure that is updated, may not be relevant for a particular patient, and may not have to be stored associated with that patient.

In an embodiment, the health database system may store medical information for multiple patients. For example, this works well in the form of a cloud solution. For example, the health database may receive medical information from multiple data sources and store them in association with the patient for whom the information is relevant. For example, the health database system may receive a care procedure, or a care procedure update and search through the multiple patients for whom the information is relevant. On the other hand, a health database could be a local health database storing only information for one or for few persons.

There are various relevancy determining algorithms that may be executed by the health database system to determine relevance of a medical update for a particular patient. For example, the health database may determine relevancy from metadata associated with the medical update, e.g., a name, an identifier. For example, a tag, such as a syndrome, medical condition, etc., that is associated with the patient may be received associated with the medical update, e.g., comprised therein. For example, the database may store associated with the patient a list of tags, and verify if any of the received tags are comprised in the stored list of tags; if so, the medical update is considered relevant.

A data driven approach may use the new update as one of its inputs and re-generate and compare some of the outputs stored in the database. If the re-generated output is different from the stored output, this can be used as an indication of relevance for example. In addition or instead of machine learning involving natural language understanding and interpretation, this can be used to determine if an update is relevant. The system may inform a human of an update and ask for their input to aid in a relevance assessment.

For example, the medical update may be associated with a version number, and the database system may be considered to verify if a received version number is a later version of a stored version number associated with stored health information, e.g., a care procedure, or care protocol. If a later version is detected, the medical update is considered relevant.

In an embodiment, the health database system is configured to determine if new treatment steps, e.g., embodied in a care procedure, have become likely as a result of a received care protocol from a first data generation system. For example, a medical update may comprise symptoms that indicate applicability of medical update, e.g., a care procedure. If the received symptoms, match symptoms associated with the patient, the medical update may be relevant.

For example, the new treatment steps may have become likely as a result of received health data, in particular test results, from a second data generation system. For example, a second data generation system, say a laboratory, may send a health updates, that another, say, a first data generation system does not know about.

For example, the health database system may be configured for sending a warning message to a further data generation system determined as relevant for the further treatment steps, wherein the further data generation system may be different from the first and second data generation systems.

For example, the health database system may be configured to determine, say, on the basis of local, private health data, say, obtained from a smartwatch, biosensor, or the like, and, say, a care procedure, or other medical information, that further treatment, possibly including further testing, is likely. As a result the health database system may send a warning to a health practitioner, e.g., to set an appointment or for a second opinion, etc.

Received new information, may be in the form of protocols, but also in the form of protocol steps, care procedures, or new data. This new data may be linked to a patient condition, but does not need to be patient data. For example, information about a new gene mutation or gene variant may be relevant for a patient, if the patient is known to have a condition affected by this gene mutation or gene variant.

A data generating systems may be a hospital, a laboratory, a private sensor, etc. A data generating systems may be a source of information, e.g., a publication, a clinical trial, etc. A data generating systems may comprise the output generated by updated processing algorithms, e.g., an AI medical system.

For example, in an embodiment, the health database system may be configured to retrieve from the database a first diagnosis based on first medical data and a first protocol according to which the first diagnosis was made. A medical update may comprise a second protocol for making a second diagnosis. The second protocol may be an update of the first protocol, e.g., as indicated by a version control system, but this is not necessary. Differences between the first protocol and the second protocol may be determined, and a likelihood may be assessed, e.g., estimated, that a second diagnosis based on the second protocol and the medical data is different from the first diagnosis. For example, the first and second protocol may be protocols to diagnose the same medical condition.

Generally speaking, receiving a medical update may have a knock-on effect, e.g., as a result of protocol update, data update, or interpretation update. That is, receiving new health data or care procedure may have further implications, for example, they may require a reinterpretation of previous data. Note that there can be more than one protocol which may have been followed. Some protocols may be focused on data collection, while others on treatment, and others and diagnosis, and so on. In the health database, a medical information may be linked to a care procedure. Note that the links between medical information and care procedures need not be one to one. A medical information may be linked to multiple care procedures. Even, when a protocol remains the same, the patient data may have been updated. For instance new patient data may have been collected, or the interpretation of the data may have been changed.

If a medical update is determined to be relevant for a particular patient, then the health database system may be configured to inform health care personnel associated with the determined patient, and/or the patient him/herself, of the determined relevancy of the medical update.

In an embodiment, the health database system is configured to compute a risk score for a patient and to update said risk score if new health data and/or a new care procedure is received for said patient, the risk score indicating a risk that health data associated with the patient have become unreliable as a result of the new care procedure. A warning may be raised if said risk score increases by more than a predetermined threshold. The threshold may be determined empirically. For example, the threshold may be tuned so that the number of warnings is below a predetermined level.

The risk score may indicate the likelihood that some patient data becomes unreliable, unusable, misleading and/or wrong, based on the medical update. In other words, as a result of receiving new information, which may be in the form of patient data, protocol, guidelines, or in general information linked to patient data, some information stored in the health database system, e.g., in the form of patient data, may need to be updated. For example, either new patient data may be collected, or new computations performed, e.g., new data analyses.

Note that a medical update may comprise a guideline update. Normally, when a guideline is updated, hospitals decide based on new guidelines how to update their protocols. In some cases, these protocol updates can be late, or may not be made at all. To act early, the system may use the guideline updates. For example, the same or similar procedure may be followed as if an update was received for a care procedure or protocol derived from the previous version of the guidelines.

A patient risk value may also be used to determine an evaluation frequency of health data based on the calculated risk value. The evaluation may be performed with the determined frequency. The relation between risk score and evaluation frequency can be modelled using a mathematical function. For example, a linear or logarithmic function can be used. In one example, an increase in risk value can increase the evaluation frequency.

The health database system may be configured to receive from the multiple data generating systems medical information comprising at least health data and one or more care procedures, one or more of the medical information comprising a link to an earlier received medical information on which it is dependent. If a medical update is received for a first medical information, it is verified if the database comprises a second medical information that is recorded as dependent upon the first medical information. If a dependent second medical information is identified a resolution algorithm may be performed. For example, the resolution algorithm may send a warning. For example, the resolution algorithm may verify if second medical information is likely to change as a result of the medical update.

For example, the database may use a directed a-cyclic graph as a suitable data structure. A dependency link may be inserted by the system automatically. For example, if a medical action, e.g., a treatment or test, is selected from a care procedure, the dependency may be inserted automatically. A health care provider (HCP) may also insert a dependency manually. A knock-on effect may be considered in by following not just paths of dependency links of length 2, but possibly of longer length as well. For example, a third medical information may depend on a second medical information which may depend on a third medical information. If the latter is updated, the first medical information may need to be reconsidered.

The resolution algorithm may consider the likelihood that reconsidering an element may lead to a different outcome. For example, a diagnosis that is supported by other elements that were not updated may not need to be reconsidered. For example, the age of the elements may be taken into account. A resolution algorithm may be an expert system, or a machine learnable system, e.g., a decision tree.

The non-limiting text below, shows additional embodiments, refinements, features and to illustrate possible embodiments.

Frequently there are new findings which can alter protocols, e.g., care plans or even guidelines. For example, what genes to test for, etc. There may be a need to reappraise historic data, e.g., to re-score risk profiles or triage choices, etc. In order to respond correctly to updates in protocols, the following elements may be considered. Patient PHR which holds variety of data related to, e.g., an oncology case. For example, comprising imaging, genomics, vital signs, lab values, and the like. Data is preferably stored in a secure storage. For example, in a distributed ledger, or a database maintained by a trusted third party, etc., to avoid tampering. Optionally the data can be stored by each care provider and an intermediary service that takes care of communicating with these. For example, the system may be configured to communicate only the relevant information to a patient. New clinical insight may be gained, e.g., a new risk score/ triage score or updated gene annotation, and the like, possibly triggered by a HCP message or a central message, etc.

Database update may be performed, e.g., by determining if the new insight relates to an entry in the database. Furthermore, the database may determine what knock-on updates are required as a result of new insight. For example, an update of a diagnosis which has not yet resulted in treatment may have to be reconsidered. In an embodiment, the system is configured to react to protocol updates. Note that this is advantageous also in situations where patients are in a non-centralized system.

Fig. 2a schematically shows an example of an embodiment of a health database system. Shown in the Figs. are data elements stored in a PHR as it moves through a distributed healthcare ecosystem. Fig. 2a shows a centralized database 260 storing relevant care procedures, care protocols, e.g., comprising care procedures, and centralized guidelines. The PHR is implemented here as a distributed ledger 270. The distributed ledger 270 may store medical information, e.g., associated with a patient. A maintainer may be configured to maintain the distributed ledger. The distributed ledger comprises records that contain information for storage. A record, except a so-called genesis block, points to one or more other, e.g., earlier records-typically a single block, forming a graph structure, typically a linear graph structure. To create a new block some qualification must often be met, e.g., a proof of work, proof of stake, or the like. A maintainer has a local copy of the database, but keeps this synchronized with other maintainers. There is a rule to decide on conflicts, typically, the larger ledger, or longer chain, is authentic. A maintainer may comprise a so-called miner, especially if the distributed ledger is of proof-of-work type. However, in an embodiment, the maintainers comprise additional functionality, e.g., responding to data queries. In an embodiment, the health database system has a local copy of the database, which is periodically updated to the database of one of the maintainers.

A maintainer may be combined with a health database system. The maintainer cooperates with other maintainers to keep the database up to date.

Fig. 2a shows three records, or blocks: block 271, block 272 and 273. The blocks contain information, e.g., information 271.1 - 273.3. Part of the information is related to the application, e.g., contain medical information. Part of the information is related to the distributed ledger. For example, information 271.3 points to block 272. Information 272.3 points to a yet prior block, and so on.

Using a distributed ledger has various advantages. There is no need for a central party that controls which copy of the database is the authentic one. For example, a hospital may store information in the ledger, but does not need to have its own maintainer, although it could have. Indeed, every health database system ledger could be maintained by different maintainers, none of which need to be associated with any hospital or site. Note that a so-called private distributed ledger may be used, without public access. Suitable distributed ledgers include blockchain, ether, directed acyclic graph based distributed ledgers, e.g., Tangle. Although a distributed ledger is advantageous, in an embodiment, a ledger may be replaced by a local database, a non-local database, e.g., in the cloud, a private database, e.g., restricted to one patient, and a shared database, e.g., storing information pertaining to multiple individuals.

Fig. 2b schematically shows an example of an embodiment of a health database system. Fig. 2b shows three databases: databases 221, 222 and 223. Database 221 may be a personal health record database (PHR). Database 222 may be a local hospital database, e.g., a protocol database, possibly with version control. Database 223 may be a database storing medical guidelines, e.g., before they are applied in the form of care protocols or care procedures. Other databases may be relevant, e.g., an EMR of the database. Shown in Fig. 2b are four data generating systems: a hospital 231, an imaging center 232, a biopsy center 233, and a GP surgery 234. In an embodiment, all data that is relevant for a particular patient is stored in PHR 221, e.g., health data coming from the data generating system, or care procedures, care protocols or guidelines or the like coming from the databases 222 and/or 223.

Interestingly, an end-user may be in control of the data. The end-user is typically a patient, though this is not necessary, it could be a family member, the care physician handling the case, or the like. For example, this could be a GP in case a patient is under remission or under active surveillance. Desirability of updating the database may depend on various factors, e.g., will updating be advantageous during therapy, it may not be desired in a palliative situation, it may depend upon how much difference the change will make to outcomes, etc.

In this example the PHR is in the form of a distributed ledger. For example, different steps in the patient treatment may be recorded in subsequent blocks. In general, the protocols will be available in a hospital/diagnosis center-controlled database 260. These will be updated frequently, typically daily or weekly, by the hospital or diagnosis center. Potentially all hospitals and diagnosis centers may update their own protocols. In this distributed system there is a desire to automate a trigger to request for a ledger update. For example, there may not be a healthcare professional (HCP) who will have the complete overview and hence it may not be feasible for a doctor to do this patient by patient. Such a trigger may be generated by the health database system.

In an embodiment, relevant guidelines and protocols are stored in the PHR, as a local copy. In an embodiment, a care procedure, protocol of guideline applies not to a specific patient, but to a class of patients, e.g., those diagnosed with a particular condition, or the like. The health database is configured to determine the relevancy of medical updates to a particular patient and to store them in association with the corresponding patient.

A suitable storage approach would be a database with version control - although any similar structure, preferably with suitable security, can be used. If the current protocols are a part of the PHR, this will make triggering the ledger update easier as the request then becomes only an external trigger to update a protocol in the protocol storage database in the PHR. This trigger then internally causes the ledger to update.

The health database system may also store how a particular care procedure is applied to a particular patient. Because protocols are written in a general manner to fit a population of patients, during the clinical practice, it is up to the clinicians how a protocol will be interpreted and applied. Steps can be skipped, or altered compared to what is written in the protocol documents. Hence, capturing and storing how a particular protocol is being applied to a particular patient is advantageous.

Different applications of the protocols may be indirectly stored in EMRs (Electronic medical records) of the patient. The EMR may also capture patient vitals, lab data, and so on. In some cases, using the EMR data the relevance of medical updates could be deduced, in practice this may be impossible, since the EMR only stores data from one data source, e.g., the hospital corresponding to the EMR.

For example, to respond to a protocol update, a protocol update may flag the patient cohort that needs to have this update added. The system may then proceed to incorporate the update into the PHR and further process the consequences as described herein.

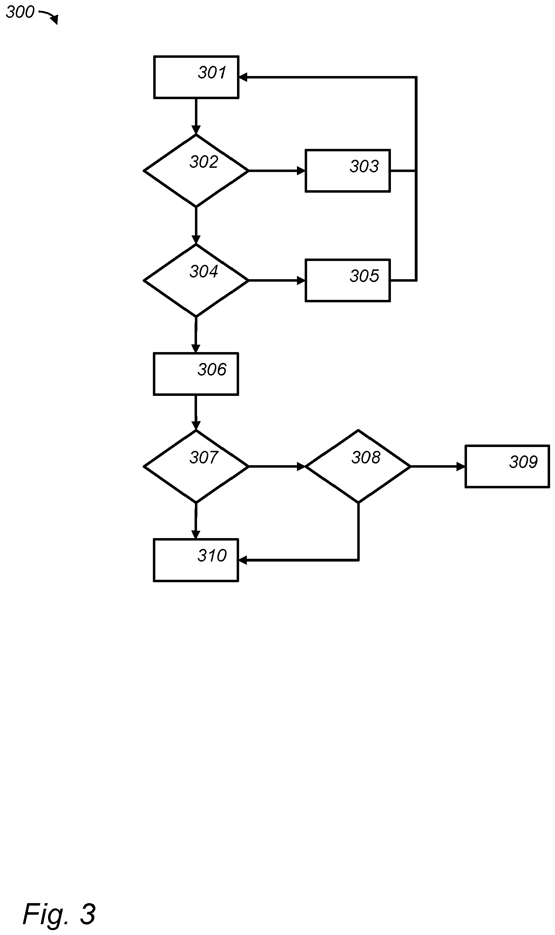

Fig. 3 schematically shows an example of an embodiment of a health database method 300, in particular one approach to updating the PHR. Shown in Fig. 3, is receiving a protocol update 301. Part 302 comprises determining if the protocol is related to a medical condition of a patient. If not, the method continues with the no-action part 303. Part 304 comprises determining if the update is sufficiently significant for a PHR update. For example, an update that merely comprises corrected typo's may not merit updating the PHR. If the update is not sufficiently significant, the method continues with the no-action box 305.

If an update is found that is relevant and significant for a patient, then the corresponding patient's PHR is updated, in parts 306. Part 307 comprises determining if the medical update has a knock-on effect on other PHR entries, in particular for this patient. For example, a knock-on effect may be that previous interpretation, treatment plan, or the like, needs reevaluation in light of the medical update. If such a need is found, the corresponding parts of the PHR may be updated as well. Other parts of the PHR are updated in part 310; for example knock-on effects may be updated in part 310.

In either case, regardless of whether a knock-on effect was found, it is determined in part 308 if a HCP, e.g., physician, needs to be informed about the PHR change. If so, in part 309, the HCP is informed in part 309.

After block 304 instead of directly going to block 306 for the PHR update, there may be an optional additional step of alerting an HCP, e.g., as in part 309, or instead of part 309. This is especially useful for protocol change based triggers. For instance, a protocol changes, and the system estimates a knock-on effect due to the protocol update. Instead of changing the PHR entries directly, a clinician may be involved, new data may be collected, and/or new computations may be made. It is not necessary that a PHR is updated after which the clinician is informed. For example, the potential for a PHR knock-on effect may be sufficient to contact the clinician.

For example, an embodiment may comprise one or more of the following elements. For example,

In the first step, the system may check if the protocol is related to an illness of a patient. This may be supported in the structure of the PHR by referring the updated protocol to the database of protocols relevant to the patient - if the protocol is one that is in the database, then further action is taken.

Not all protocol updates are of sufficient importance to require a complete update of the PHR. If the significance is sufficient, the update starts, for example by updating an entry in a ledger.

Existing entries in the ledger may not be independent. For example, in typical care paths the outcome of a diagnosis usually results in a choice for a following step, e.g., further diagnoses or treatments. As such, in a following step the system may consider if the initial ledger update has knock-on effects resulting in changes elsewhere in the ledger. As an example, a protocol change may result in a change in a first risk score from a diagnosis, which may have an effect on future assessments of further risk scores which depend upon the first risk score.

Once the PHR has been fully updated, the system may assess which physicians or institutions needs to be alerted of the changes and sends a notification to the relevant physician or institution that a significant update has occurred and that further action is required, e.g., a recall of the patient.

In an embodiment, database records are linked, one or more of multiple records in the database comprising a link to one or more other records in the database. The link indicating a dependency. After updating a record in the database, the links in the record are followed to determine if the linked other records need updating as well. The links may be followed recursively.

It is desirable to re-evaluate the effect of protocol changes on personal health efficiently. For example, protocol updates may be categorized in terms of their significance, and determine reevaluation frequency of personal data based on the calculated significance. For example, one may evaluate PHR with respect to the protocol update and calculate a significance number. Additionally, clinicians' input can be used too. Significance calculation may be done by using the received data and possibly using selected data stored in the database. Accordingly, significance calculation is efficient as no external data needs to be requested. The re-evaluation frequency of PHR may be set according to the significance number. For example, the mapping from significance to frequency may use a look-up table.

Re-evaluation according to a frequency, e.g., to assess the effect of a change, such as a protocol change, may be needed because a PHR may be continuously changing and growing, e.g., due to new incoming patient data and health information becoming available or accessible. For example, vital sign monitoring may frequently add information to the PHR. For example, care protocol may change frequently, e.g., to reflect new medical research.

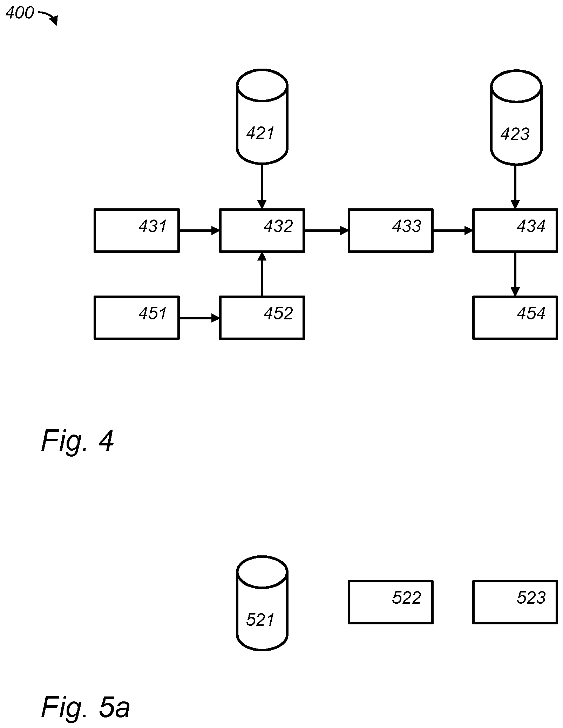

Fig. 4 schematically shows an example of an embodiment of a health database system 400. The evaluation of protocol change may first use a smaller set of PHR data, and depending on the outcome of the first evaluation, either determining the next evaluation moment or evaluating the change using all PHR data.

Shown in Fig. 4 is a part 432 configured for protocol update significance calculation. Part 432 uses input from one or more of database 421, e.g., a PHR, HCP input 431, and a protocol update 452. Protocol update 452 may be an update of protocol 451.

Part 433 may be configured for PHR analysis frequency calculation. For example, the PHR may be re-evaluated only at frequencies determined from the significance of the protocol update. For example, part 434 may be configured for protocol effect calculation. Part 434 may use as an input PHR update 423. If the significance is sufficiently high, then in part 454, an HCP, e.g., a clinician is informed, e.g., to change treatment if necessary.

In some cases, a patient is under active or watchful surveillance. For instance, in prostate cancer this is a treatment regime when the prostate cancer is not considered to be so threatening that an intervention outweighs the risk of not intervening. Also in lung cancer clinicians may make such a decision when a patient is elderly and the lesions are still so small that the risk of surgically removing the tumors/metastases are too high given the patient's age.

In such cases, new insights, such as for lung cancer new genomic insights, may suggest that a new treatment, e.g., a certain target drug or immuno-therapy may be effective. In such a case, a PHR user, for instance the GP of the patient, or his/her oncologist, or the patient themselves may update the patient's PHR to see whether for a particular patient based on earlier genomic analysis of the patient's tumor a patient would qualify for this treatment. Possibly, some patients will qualify where others will not. For example, immuno-therapy is known to work for a selected group of patient and not at all in many other patients. Alternatively, further understanding of the patient groups for which an immuno-therapy may be effective may trigger an oncologist to trigger an update to a PHR of a patient, which the patient may check and accept. In such a way, a distributed PHR may become an efficient route for an oncologist to reach out to his past patients without having to call all his patients, or to manually go through patients record to check which of his patients would qualify for a new drug based on some special and previously insignificant mutation detected in a previous sequencing analysis of tumors. For example, based on new drug targets, gene variants, which were previously unknown to play a role or were un-targetable, may become relevant. This reanalysis can be performed on the previous sequencing analysis of the tumors.

Updates of a protocol result may cause an updating of a PHR. For example, these cases may include protocol updates related to imaging, genomics, lab tests, vital signs, and the like. Other protocol updates may also cause an update.

In an embodiment, a second institute may reassess a diagnosis from first institute. For example, a patient, who stores its clinical data in a PHR may contact to a second institute who reassess diagnosis from a first institute, e.g., an image scan from a scanning center, re-assessed at a hospital, etc. The first and second institutes may use different protocols for the same diagnosis. Fig. 5a schematically shows an example of an embodiment of a health database system. Shown in Fig. 5a is a PHR 521, an institute 1 protocol 522 and an institute 2 protocol 523.

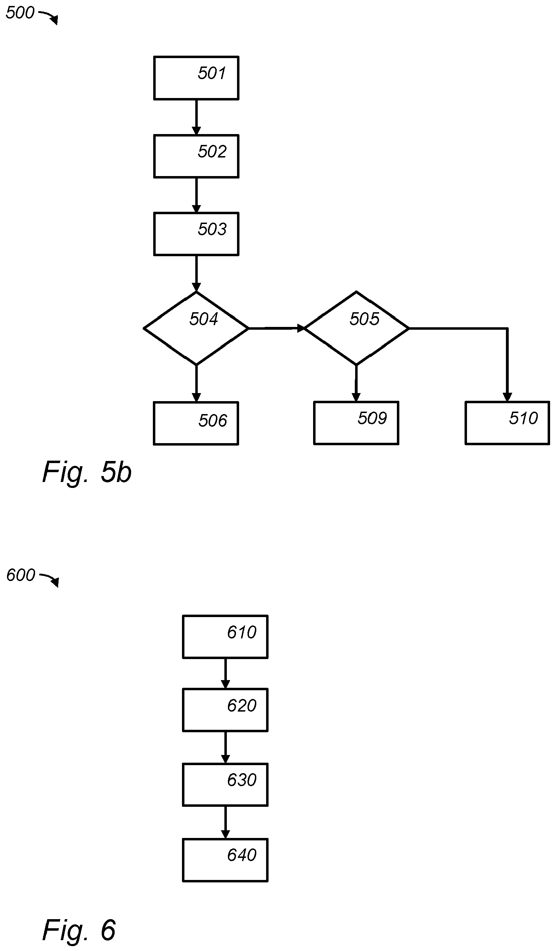

Fig. 5b schematically shows an example of an embodiment of a health database system 500. Fig. 5b shows in part 501, a patient attending institute 1 and is diagnosed with protocol 1. In part 502, protocol 1 is added to the patient's PHR, e.g., by a health database system. In part 503, the patient attends institute 2 for diagnosis. In part 504, a health database system determines if institute 2 and institute 1 use the same protocol. If so, in part 506 it is determined that a repeating of, e.g., diagnosis by institute 2 is not sensible; as the same care protocol is followed, the same diagnosis would result.

If institute 2 were to carry out the first diagnosis following protocol 1 as was done by institute 2, there is no requirement to carry out the first diagnosis again. Institute 2 would have reached the same conclusion as institute 1 and can proceed with their follow-up diagnosis.

If institute 2 would carry out the first diagnosis following their own procedure (protocol 2), then protocol 2 may be added to the PHR as it is of relevance for further processing. Note that if the protocol was applied to the patient, then it may be in the PHR, regardless if it is the same as protocol 1.

On the other hand, in part 505, if the protocols are not the same, it is determined if the second protocol would result in the same diagnosis as the first protocol.

For example, a conflict orchestrator algorithm may decide whether Institute 2 would have reached a different conclusion as institute 1; for example they would have assigned a different risk score. If this is the case then the orchestrator can propose that Institute 2 repeats the first diagnosis according to protocol 2.

If the same diagnosis would result, then in part 510 this is flagged, and re-diagnosis is not needed. If the two protocols were to come to a different conclusion, then in part 509 this conflict or discordance is flagged, and possibly re-diagnosis is scheduled.

Below a number of situations are provided which may be processed to advantage with a system according to an embodiment.

Situation A. Patient 1 lives in Boston and is diagnosed with stage I non-small cell lung adenocarcinoma in 2021 at the age of 62. A lobectomy is performed to surgically remove the tumor, followed by adjuvant chemotherapy consisting of cisplatin and vinorelbine. Molecular testing of the resected tumor using the MSK-IMPACT gene panel reveals a mutation count of 4. These data are recorded in Patient 1's PHR; For example, the data is recorded in a database storage for storing at least health data in association with patient 1. As of May 2021, 3 of the 4 mutated genes are annotated, e.g., by OncoKB (Human Genetic Variant Database), including an EGFR mutation. In May 2021, the EGFR mutation is annotated at Level 4 ("Compelling biological evidence supports the biomarker as being predictive of response to a drug"). Since at the time none of the mutations were annotated with a Level 1 ("FDA-recognized biomarker predictive of response to an FDA-approved drug in this indication"), or Level 2 ("Standard Care biomarker recommended by the NCCN or other professional guidelines predictive of response to an FDA-approved drug in this indication") no targeted therapy is prescribed by the Boston oncologist. Patient 1 moves to Austin in 2022. In 2023, the NCCN and OncoKB re-evaluates the annotation of EGFR L747, raising the status from Level 4 to Level 2. Patient 1's PHR is updated to include the new Level 2 status of EGFR L747. The PHR determines that the received medical update is relevant for the patient, and indicates to the new Austin oncologist treating Patient 1 that, based on the new Level 2 status of the EGFR L747P mutation, that he/she should administer afatinib (a targeted tyrosine kinase inhibitor) to Patient 1.

Patient 1's oncologist in Austin want to confirm findings and orders a liquid biopsy test targeting the EGFR, TP53, and PIK3CA genes based on Patient 1's PHR. This test reveals relapse of patient 1 and circulation of the EGFR L747P mutation.

Situation B: Patient 2 lives in Miami and is diagnosed with stage IIIC high-grade serious ovarian cancer in 2021 at the age of 57. Neoadjuvant chemotherapy consisting of cisplatin and paclitaxel is performed before surgery. Molecular testing of the biopsy tissue with the FoundationOne CDx test reveals a mutation count of 38. As of May 2021, 3 of the 38 mutations were annotated by OncoKB. No genomic testing of the tumor biopsy was performed, thus no HRD (Homologous recombination deficiency) was detected, and no targeted therapy was prescribed due to a lack of obvious candidate variants driving HRD (such as BRCA1/2). In April 2022, the FDA announces expansion of the indication of niraparib to include pathogenic mutations in the DNA-damage response gene RAD51. Given the clear pathogenic Q145^{∗} mutation of RAD51, Patient 2's PHR is updated to include the new Level 1 ("FDA-recognized biomarker predictive of response to an FDA-approved drug in this indication") status of RAD51. Patient 2 does not consult her PHR and takes no action. Patient 2's oncologist in Miami is notified of the new FDA approval and in May 2022. She generates a list of all her living ovarian cancer patients who had received a molecular test revealing a pathogenic variant in RAD51, including Patient 2. Patient 2's oncologist phones her and suggests that, given the new FDA approval for niraparib and Patient 2's pathogenic variant in RAD51, Patient 2 commence treatment with niraparib.

According to an embodiment, Patient 2's PHR could be configured to determine that the niraparib update is relevant for Patient 2, as it she is diagnosed with ovarian cancer and had a molecular test revealing a pathogenic variant in RAD51. Accordingly, the HCP could be signaled to prompt action.

Situation C. Patient 3 lives in Denver and has a history of anemia. She is diagnosed with stage I invasive breast carcinoma in 2015 at the age of 80. Surgical mastectomy and radiation therapy is performed. Whole genome and RNA sequencing of the resected tumor tissue is performed as part of a clinical study sponsored by her oncologist and the attending hospital. A mutation count is calculated at 15, as well as 58 copy number alterations including a deep deletion of RAD51. The parallel RNA sequencing reveals a very low (9%) expression of RAD51.

In April 2022, the FDA announces expansion of the indication of niraparib to include pathogenic mutations in the DNA-damage response gene RAD51. Given the deletion of RAD51, Patient 3's PHR is updated to include the new Level 1 ("FDA-recognized biomarker predictive of response to an FDA-approved drug in this indication") status of RAD51. Patient 3 contacts her oncologist who has also updated her list of breast cancer patients who had molecular testing revealing pathogenic RAD51 variants, including Patient 3. Patient 3's PHR notes her most recent blood test reveals a low red blood cell count. Patient 3's oncologist decides that given Patient 3's advanced age of 87 coupled to her anemia that commencing a course of niraparib would cause more pain than gain. Patient 3's oncologist removes the niraparib recommendation from Patient 3's PHR and updates her own records to remove Patient 3 from her list of breast cancer patients with pathogenic RAD51 mutations.

Situation D. Patient 4 lives in Kansas City and is diagnosed with stage II non-small cell lung adenocarcinoma in 2021 at the age of 67. A lobectomy is performed, followed by chemotherapy consisting of cisplatin and paclitaxel, followed by radiation therapy. Molecular testing of the tumor biopsy tissue reveals a mutation count of 2, including the actionable L858R hotspot mutation in the EGFR gene. Based on the presence of the EGFR mutation, erlotinib is prescribed to Patient 4 as maintenance therapy, and he goes for monthly liquid biopsy PCR tests to test for the acquired EGFR T790M resistance mutation.

In 2023, Patient 4 moves from Kansas City to Seattle where he continues the liquid biopsy PCR tests. In 2024, the FDA approves liquid biopsy tests for detecting acquired resistance to erlotinib to include variants in BRAF. Patient 4's oncologist in Kansas City initiates an update of all PHRs of her patients on erlotinib as maintenance therapy. Patient 4 receives the update on his PHR in Seattle, which recommends that he begin simultaneously testing for acquired resistance mutations in BRAF as well as EGFR. Patient 4 brings his updated PHR to the testing center, where they adjust the amplicons they sequence to include BRAF. In 2025, Patient 4 receives a PCR test result for BRAF detecting a V600E mutation. The PCR test result suggesting that Patient 4 may have acquired resistance to erlotinib is updated in his PHR as well as his oncologist's records in Kansas City. The PHR determines that this is relevant and signals this with Patient 4's oncologist. Patient 4's oncologist contacts him in Seattle and recommends that he go for a CT scan as soon as possible. If he is unable to get to Kansas City, she recommends a colleague of hers in the Seattle area. Patient 4's oncologist also recommends that he switches maintenance therapy from erlotinib to gefitinib.

Situation E. Patient 5 lives in New York City, has a familial history of cancer, and was diagnosed with stage IIIB prostate cancer in 2015 at the age of 62. The initial diagnosis followed a routine blood test reveals a PSA level of 15ng/mL. A radial prostatectomy is performed in 2015 followed by radiation therapy. Following treatment and for the next four years, Patient 5's PSA levels remain within the range of 1-10ng/mL, with an upward trend noted in the tests performed in 2019.

Given Patient 5's family history of cancer, germline molecular testing of select genes is performed in 2016 and reveals an inherited frameshift mutation in the BRCA2 gene, Y949Lfs^{∗} 10. In January 2020, Patient 5 complains about extreme fatigue and pain in his hips. A CT scan reveals the prostate cancer has metastasized to his pelvic bone. A blood test revealed a rise of PSA levels to 18ng/mL consistent with the upward trend noted in 2019. Patient 5 is diagnosed with castration-resistant prostate cancer (CRPC). In February 2020 Patient 5 receives taxane chemotherapy treatment in the form of docetaxel. In May 2020, the FDA approves the PARP inhibitor rucaparib for men with metastatic CRPC and a deleterious BRCA2 mutation. Patient 5 is determined as fitting all the criteria for rucaparib administration, and is notified on his PHR. His oncologist in NYC is also notified of her patients with CRPR who now qualify for rucaparib. The NYC hospital contacts Patient 5 to alert him of this new qualification, he has already taken the initiative and contacted his oncologist for the rucaparib prescription.

Situation F: Patient 6 is a 45-year-old woman with no history of cancer who lives in London. In 2016 at the age of 40, Patient 6 had a single mammogram as part of routine breast cancer screening. The mammogram results in 2011 did not identify any suspicious lesions, however, using the BI-RADS scoring system, the mammogram results did establish that her breast tissue density is classified at the D level (D = extremely dense). Patient 6, having found her initial mammogram to be a painful experience and believing that only women over the age of 50 need routine mammograms, did not go back for further screening. In 2021, an oncologist joined a collaborative effort with the radiology department of her hospital to perform a retrospective analysis on healthy women, identifying those who had been classified with C-D scoring of breast tissue density according to BI- RADS and who had not returned for additional screening. The oncologist ranked all the women in the study according to age and BI-RADS breast tissue density score. The oncologist contacted all the women in her study who she found to have C-D breast tissue density scores, were over the age of 40, and who were not in a routine screening program by sending a medical update. The recommendation received by Patient 6 on her PHR stated that, given her high-risk breast tissue density score of D coupled to her age, that she begin routine annual breast cancer screening.

Situation G: Drug repurposing (alternatively called "new uses for old drugs") is a strategy for identifying new uses for approved or investigational drugs that are outside the scope of the original medical indication. An example of Drug repurposing is Metformin. Originally Metformin was approved by the FDA in 1994 for treating obese type 2 diabetes. Long-term metformin treatment also showed lower risk of breast cancer in women with type 2 diabetes. Currently, many clinical trials are investigating the anticancer activity of metformin, using doses typically used for diabetes, for cancer therapy. Trails advanced to Phase III and Phase IV, investigating the therapeutic potential of metformin in oral, prostate, breast, endometrial, and pancreatic cancers.

Results from clinical trials using Metformin will lead to an update of the PHR to include this treatment as anti-cancer therapy option.

Fig. 6 schematically shows an example of an embodiment of a health database method 600. Method 600 may be computer implemented and comprises:
- receiving (610) health data relating to a patient from multiple data generating systems,
- receiving (620) medical updates from the multiple data generating systems,
- storing (630) at least health data in association with a patient,
- determining (640) if a received medical update is relevant for the patient, and, if so, store the medical update in the database storage associated with the determined patient.

Many different ways of executing the method are possible, as will be apparent to a person skilled in the art. For example, the order of the steps can be performed in the shown order, but the order of the steps can be varied or some steps may be executed in parallel. Moreover, in between steps other method steps may be inserted. The inserted steps may represent refinements of the method such as described herein, or may be unrelated to the method. For example, some steps may be executed, at least partially, in parallel. Moreover, a given step may not have finished completely before a next step is started.

Embodiments of the method may be executed using software, which comprises instructions for causing a processor system to perform method 600. Software may only include those steps taken by a particular sub-entity of the system. The software may be stored in a suitable storage medium, such as a hard disk, a floppy, a memory, an optical disc, etc. The software may be sent as a signal along a wire, or wireless, or using a data network, e.g., the Internet. The software may be made available for download and/or for remote usage on a server. Embodiments of the method may be executed using a bitstream arranged to configure programmable logic, e.g., a field-programmable gate array (FPGA), to perform the method.

It will be appreciated that the presently disclosed subject matter also extends to computer programs, particularly computer programs on or in a carrier, adapted for putting the presently disclosed subject matter into practice. The program may be in the form of source code, object code, a code intermediate source, and object code such as partially compiled form, or in any other form suitable for use in the implementation of an embodiment of the method. An embodiment relating to a computer program product comprises computer executable instructions corresponding to each of the processing steps of at least one of the methods set forth. These instructions may be subdivided into subroutines and/or be stored in one or more files that may be linked statically or dynamically. Another embodiment relating to a computer program product comprises computer executable instructions corresponding to each of the devices, units and/or parts of at least one of the systems and/or products set forth.

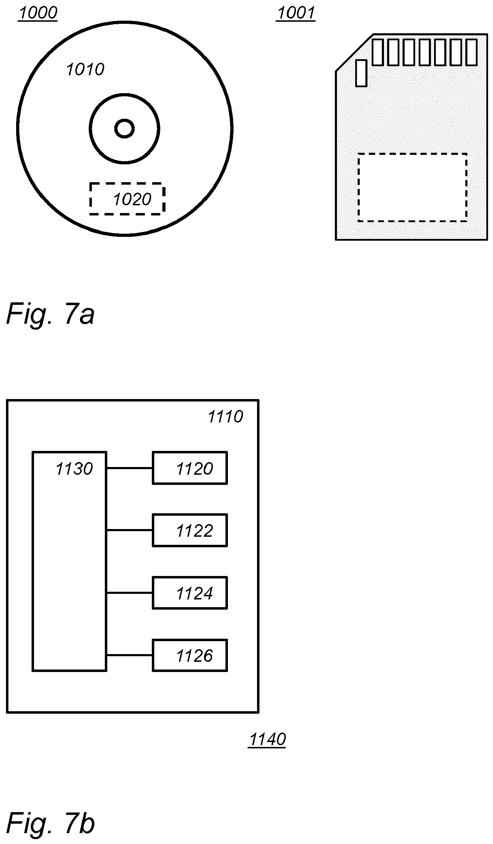

Fig. 7a shows a computer readable medium 1000 having a writable part 1010, and a computer readable medium 1001 also having a writable part. Computer readable medium 1000 is shown in the form of an optically readable medium. Computer readable medium 1001 is shown in the form of an electronic memory, in this case a memory card. Computer readable medium 1000 and 1001 may store data 1020 wherein the data may indicate instructions, which when executed by a processor system, cause a processor system to perform a health database method, according to an embodiment. The computer program 1020 may be embodied on the computer readable medium 1000 as physical marks or by magnetization of the computer readable medium 1000. However, any other suitable embodiment is conceivable as well. Furthermore, it will be appreciated that, although the computer readable medium 1000 is shown here as an optical disc, the computer readable medium 1000 may be any suitable computer readable medium, such as a hard disk, solid state memory, flash memory, etc., and may be non-recordable or recordable. The computer program 1020 comprises instructions for causing a processor system to perform s health database method.

Fig. 7b shows in a schematic representation of a processor system 1140 according to an embodiment of a health database system. The processor system comprises one or more integrated circuits 1110. The architecture of the one or more integrated circuits 1110 is schematically shown in Fig. 7b. Circuit 1110 comprises a processing unit 1120, e.g., a CPU, for running computer program components to execute a method according to an embodiment and/or implement its modules or units. Circuit 1110 comprises a memory 1122 for storing programming code, data, etc. Part of memory 1122 may be read-only. Circuit 1110 may comprise a communication element 1126, e.g., an antenna, connectors or both, and the like. Circuit 1110 may comprise a dedicated integrated circuit 1124 for performing part or all of the processing defined in the method. Processor 1120, memory 1122, dedicated IC 1124 and communication element 1126 may be connected to each other via an interconnect 1130, say a bus. The processor system 1110 may be arranged for contact and/or contact-less communication, using an antenna and/or connectors, respectively.

For example, in an embodiment, processor system 1140, e.g., the health database system may comprise a processor circuit and a memory circuit, the processor being arranged to execute software stored in the memory circuit. For example, the processor circuit may be an Intel Core i7 processor, ARM Cortex-R8, etc. The memory circuit may be an ROM circuit, or a non-volatile memory, e.g., a flash memory. The memory circuit may be a volatile memory, e.g., an SRAM memory. In the latter case, the device may comprise a non-volatile software interface, e.g., a hard drive, a network interface, etc., arranged for providing the software.

While device 1100 is shown as including one of each described component, the various components may be duplicated in various embodiments. For example, the processor 1120 may include multiple microprocessors that are configured to independently execute the methods described herein or are configured to perform steps or subroutines of the methods described herein such that the multiple processors cooperate to achieve the functionality described herein. Further, where the device 1100 is implemented in a cloud computing system, the various hardware components may belong to separate physical systems. For example, the processor 1120 may include a first processor in a first server and a second processor in a second server.

It should be noted that the above-mentioned embodiments illustrate rather than limit the presently disclosed subject matter, and that those skilled in the art will be able to design many alternative embodiments.

In the claims, any reference signs placed between parentheses shall not be construed as limiting the claim. Use of the verb 'comprise' and its conjugations does not exclude the presence of elements or steps other than those stated in a claim. The article 'a' or 'an' preceding an element does not exclude the presence of a plurality of such elements. Expressions such as "at least one of' when preceding a list of elements represent a selection of all or of any subset of elements from the list. For example, the expression, "at least one of A, B, and C" should be understood as including only A, only B, only C, both A and B, both A and C, both B and C, or all of A, B, and C. The presently disclosed subject matter may be implemented by hardware comprising several distinct elements, and by a suitably programmed computer. In the device claim enumerating several parts, several of these parts may be embodied by one and the same item of hardware. The mere fact that certain measures are recited in mutually different dependent claims does not indicate that a combination of these measures cannot be used to advantage.

In the claims references in parentheses refer to reference signs in drawings of exemplifying embodiments or to formulas of embodiments, thus increasing the intelligibility of the claim. These references shall not be construed as limiting the claim.

## Claims

1. A health database system comprising
- a data interface configured for
- receiving health data relating to a patient from multiple data generating systems, and
- receiving medical updates from the multiple data generating systems,
- a database storage for storing at least health data in association with a patient, and
- a processor configured to
- determine if a received medical update is relevant for the patient, and, if so, store the medical update in the database storage associated with the determined patient.

2. A health database system, as in Claim 1, wherein the medical update comprises a care procedure from one or more of the multiple data generating systems, a care procedure relating to multiple patients.

3. A health database system as in Claim 2, wherein receiving a care procedure comprises receiving a care procedure update, the received care procedure comprising a new version identifier, determining if the received medical update is relevant for a patient comprises verifying that the database storage stores an association between the patient and a care procedure that has a stored version identifier matching the new version identifier, wherein the health database system processor is configured to execute a version matching function to determine if the new version identifier and the stored identifier match.

4. A health database system, as in any one of the preceding claims, wherein the medical update comprises health data from one or more of the multiple data generating systems.

5. A health database system as in Claim 4, wherein receiving health data comprises receiving a health data update of a previous health data, determining if the received medical update is relevant for a patient comprises verifying that the database storage stores an association between the previous health data and a care procedure, wherein the health database system processor is configured to determine if the health data update is relevant for the care procedure.

6. A health database system as in any one of the preceding claims, comprising
- retrieving from the database a first diagnosis based on first medical data and a first protocol according to which the first diagnosis was made,
- obtaining a second protocol for making a second diagnosis,
- determining differences between the first protocol and the second protocol, and assessing a likelihood that a second diagnosis based on the second protocol and the medical data is different from the first diagnosis.

7. A health database system as in any one of the preceding claims, wherein the database storage comprises an electronic medical record (EMR), an electronic health record (EHR), and/or personal health record (PHR).

8. A health database system as in any one of the preceding claims, wherein the health data stored in association with a patient comprises one or more of
- clinical information, including, diagnosis, test results, medical images, assessments, genomic data, lab values, and vital signs.

9. A health database system as in any one of the preceding claims, wherein
- the health database system processor is configured to compute a risk score for the patient and to update said risk score if new health data and/or a new care procedure is received for said patient, the risk score indicating a risk that health data associated with the patient becoming unreliable as a result of the new care procedure,
- raise a warning if said risk score increases by more than a predetermined threshold.

10. A health database system as in any one of the preceding claims, wherein a care procedure is stored in association with a patient, as well as an application of the care procedure to said patient, obtained by applying the care procedure to the patient and specifying care procedure for the patient.

11. A health database system as in any one of the preceding claims, wherein the health data stored in association with a patient, comprises particular treatment and/or test steps that have been taken for a patient as indicated by an application of a care procedure to a patient.

12. A health database system as in any one of the preceding claims wherein the database storage comprises a distributed ledger, new tokens stored in said distributed ledger comprising health data and/or care procedures associated with a patient.

13. A health database system as in any one of the preceding claims, wherein the health database system processor is configured to
- calculate a patient risk value,
- determine an evaluation frequency of health data based on the calculated risk value
- perform evaluation with the determined frequency.

14. A health database system as in any one of the preceding claims, wherein the health database system is configured to receive from the multiple data generating systems medical information comprising at least health data and one or more care procedures, one or more of the medical information stored in the database storage comprising a link to an earlier received medical information on which it is dependent, the processor being configured to determine for a received medical update for a first medical information if the database comprises a second medical information that is recorded as dependent upon the first medical information, and if the dependent second medical information is found to perform a resolution algorithm.

15. A health database method comprising
- receiving health data relating to a patient from multiple data generating systems,
- receiving medical updates from the multiple data generating systems,
- storing at least health data in association with a patient,
- determining if a received medical update is relevant for the patient, and, if so, store the medical update in the database storage associated with the determined patient.

16. A transitory or non-transitory computer readable medium (1000) comprising data (1020) representing instructions, which when executed by a processor system, cause the processor system to perform the method according to claim 15.
